(19) Europäisches Patentamt
European Patent Office
Office européen des brevets

(11) Publication number: **0 516 157 A1**

# (12) EUROPEAN PATENT APPLICATION

(21) Application number: **92109112.0**

(51) Int. Cl.$^5$: **C07H 17/04**, A61K 31/70

(22) Date of filing: **29.05.92**

(30) Priority: **30.05.91 US 707504**

(43) Date of publication of application:
**02.12.92 Bulletin 92/49**

(84) Designated Contracting States:
**AT BE CH DE DK ES FR GB GR IT LI LU MC NL PT SE**

(71) Applicant: **Bristol-Myers Squibb Company**
**345 Park Avenue**
**New York, N.Y. 10154(US)**

(72) Inventor: **Toda, Soichiro**
**3-591-5, Ohnari-cho**
**Ohmiya(JP)**
Inventor: **Yamashita, Haruhiro**
**3-3-295, Higashi-shisui**
**Shisui-machi, Inba-gun, Chiba(JP)**
Inventor: **Naito, Takayuki**
**2657-45, Ohzenji Asao-ku**
**Kawasaki(JP)**
Inventor: **Nishiyama, Yuji**
**2-15-7-301 Kuramae**
**Taito-ku, Tokyo(JP)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Preparation of 6-0-alkylelsamicin A derivatives.

(57) This invention relates to novel elsamicin A derivatives having 6-O-alkyl substitutent, a process for producing said elsamicin A derivatives, antitumor compositions containing the same as the active ingredient, and a method for therapy using said compositions.

EP 0 516 157 A1

This invention relates to novel elsamicin A derivatives which have improved antitumor activity, lower toxicity and/or better pharmacokinetic properties, to their production, to compositions containing the same as the active ingredient, and a method for therapy using said compositions.

Elsamicin A is an antitumor antibiotic produced by cultivating an elsamicin A-producing strain of actinomycete designated strain J907-21 (ATCC 39417), or a mutant thereof. Elsamicin A exhibits antibacterial activity against aerobic gram-positive bacteria and anaerobic bacteria. It also exerts activity against various murine tumor cells including leukemia P388, lymphoid leukemia L1210, and melanotic melanoma B16 in vitro and in vivo. Konishi, et al, Elsamicins, new antitumor antibiotics related to chartreusin. I. Production, isolation, characterization and antitumor activity, J. Antibiotics, 39: 784-791, (1986); U.S. Patent 4,518,589 to Konishi, et al, issued May 21, 1985.

The structure of elsamicin A (Formula I, below) has been determined and shown to be closely related to chartreusin (Formula II, below). Sugawara, et al, Elsamicins A and B, new antitumor antibiotics related to chartreusin. II. Structures of elsamicins A and B. J. Org. Chem., 52: 996-1001, (1987).

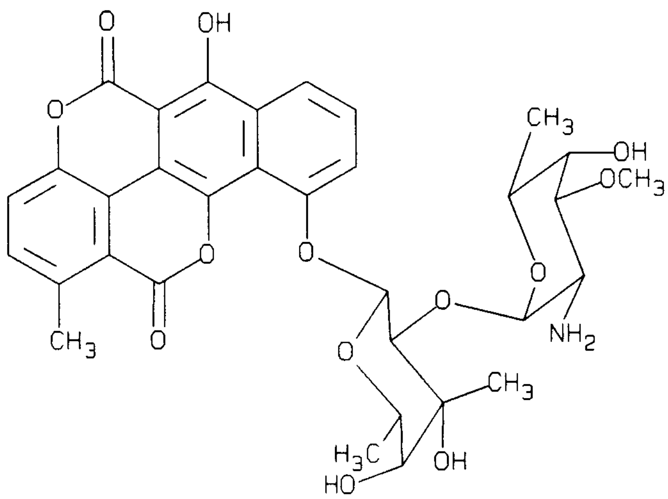

**Formula I**

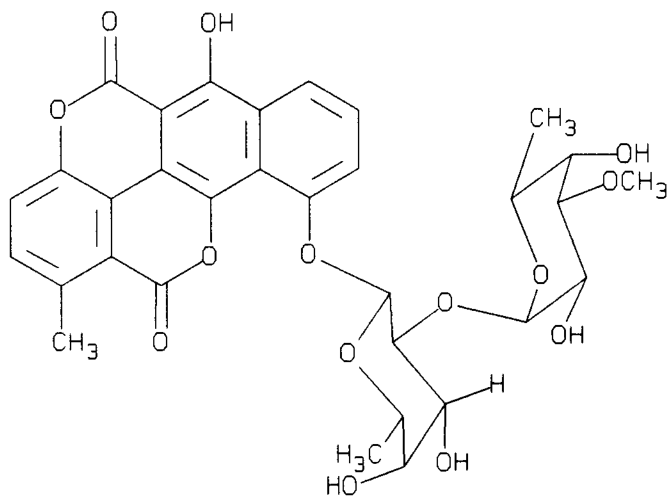

**Formula II**

Both elsamicin A and chartreusin have the same aglycone, chartarin, but the antibiotics differ in the disaccharide moiety. Leach, et al, Chartreusin, a new antibiotic produced by Streptomyces chartreusis, a new species, J. Am. Chem. Soc., 75: 4011-4012, (1953); Beisler, J.A., Chartreusin, a glycosidic antitumor antibiotic for Streptomyces, In progress in Medicinal Chemistry, Ed., G.P. Ellis and G.B. West 19: pp. 247-268, Elsevier Biomedical Press Amsterdam, (1982); Simonitsch, et al: Über die Struktur des Chartreusins I,

Helv. Chim. Acta, 47: 1459-1475, (1964); Eisenhuth, et al, Über die Struktur des Chartreusins II, Helv. Chim. Acta, 47, 1475-1484, (1964). Interconversion of both compounds by chemical process has never been reported.

In the course of chemical modification of elsamicin A, we found that alkylation on the 6-OH group of elsamicin A gave new derivatives having improved antitumor activity, lower toxicity and/or better pharmacokinetic properties.

The present invention provides new derivatives of elsamicin A which exhibit improved antitumor activity, lower toxicity and/or better pharmacokinetic properties. More particularly the present invention provides alkylation derivatives of the hydroxyl at the 6 position of elsamicin A.

This invention further provides an antitumor composition comprising, as the active ingredient, at least one member selected from the group consisting of the elsamicin A derivative of the present invention.

This invention further provides a method for therapy of cancer using the above antitumor composition.

Further provided is a process for producing the above-mentioned elsamicin A derivative.

U. S. Patent No. 4,518,589 to Konishi et al, discloses the production and isolation of the antitumor agent designated elsamicin A. (Formula I, above). The above-mentioned elsamicin A compound is the principal component of the fermentation of the elsamicin A-producing strain of actinomycete, designated strain J907-21 (ATCC 39417).

It has now been found according to the present invention that alkylation on the 6-OH position of elsamicin A gave new derivatives having improved antitumor activity, lower toxicity and/or better pharmacokinetic properties.

The elsamicin A derivatives of the present invention have the general Formula III and IV below

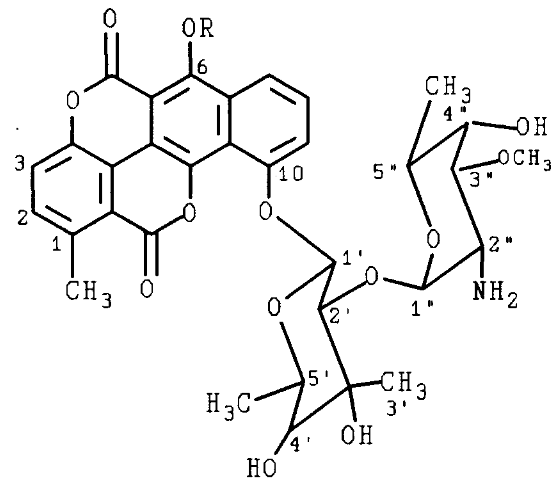

Formula III

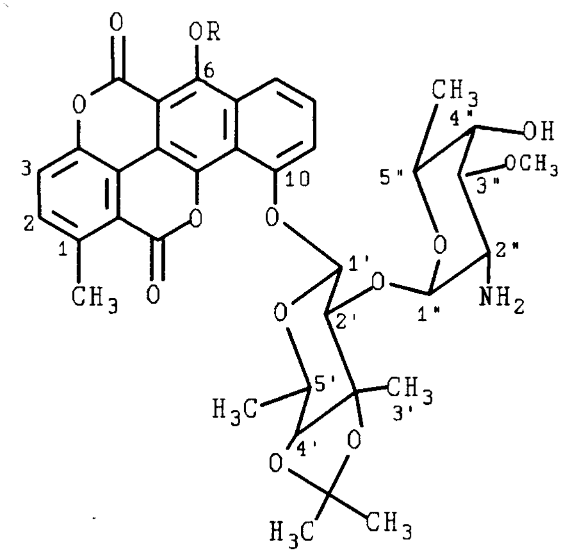

Formula IV

wherein R is straight $C_1$-$C_{10}$ alkyl, or substituted or nonsubstituted amino alkyl, preferably amino $C_1$-$C_{10}$ alkyl. Preferred substituents of the aminoalkyl group are 3-phthalimido or amino.

The term "$C_1$-$C_{10}$ alkyl" as used herein and in the claims (unless the context indicates otherwise) means branched or straight chain hydrocarbon group having 1 to 10 carbon atoms such as methyl, ethyl, propyl, isopropyl, butyl, isobutyl, t-butyl, etc.

The elsamicin derivatives of the present invention may be produced, for example by the synthetic process diagrammed in scheme 1. As shown in scheme 1, the 6-O-alkyl derivatives (7) and the corresponding 6-O-alkyl-3',4'-O-isopropylidene derivatives (8) were prepared via N-t-butoxycarbonyl (t-BOC) elsamicin (3) and/or its 3',4'-O-isopropylidene (IP) derivative (4). 6-O-Alkylation of derivatives 3 and 4 was accomplished by treatment with appropriate alkyl halides in the presence of potassium iodide (KI) and potassium carbonate ($K_2CO_3$) in dimethylsulfoxide (DMSO) to give the corresponding 6-O-alkyl derivatives 5 and 6, respectively. The t-BOC group of derivative 5 was removed to afford derivative 7 by treatment with aqueous trifluoroacetic acid (TFA). A similar treatment of derivative 6 with TFA resulted in deblocking of both t-BOC and IP groups to give compound 7. 6-O-Methylelsamicin A (7a) was also obtained by direct O-methylation of compound 1 with diazomethane in dioxane-ether.

The t-BOC group of derivative 6 was selectively cleaved by toluenesulfonic acid (TsOH) in acetone or

trifluoroacetic acid (TFA) at room temperature to afford compound 8. The phthalimido group of compound 8h was removed by treatment with hydrazine hydrate to give compound 8i, which was converted to compound 7i by eliminating the IP group.

Table 1 indicates the compounds of the present application and their respective number.

Table 1

| Compound of the present invention and their respective number | |
|---|---|
| Compound No. | Name |
| 1 | Elsamicin A |
| 2 | Chartreusin |
| 3 | 2''-N-t-Butoxycarbonylesemicin A |
| 4 | 2''-N-t-Butoxycarbonyl-3'-4'-O-isopropylideneelsamicin A |
| 5a | 2''-N-t-Butoxycarbonyl-6-O-methylelsamicin A |
| 5c | 2''-N-t-Butoxycarbonyl-6-O-n-propylelsamicin A |
| 5d | 2''-N-t-Butoxycarbonyl-6-O-isopropylelsamicin A |
| 5e | 2''-N-t-Butoxycarbonyl-6-O-n-butylelsamicin A |
| 5f | 2''-N-t-Butoxycarbonyl-6-O-n-hexylelsamicin A |
| 6a | 2''-N-t-Butoxycarbonyl-3',4'-O-isopropylidene-6-O-methylelsamicin A |
| 6b | 2''-N-t-Butoxycarbonyl-6-O-ethyl-3',4'-O-isopropylideneelsamicin A |
| 6f | 2''-N-t-Butoxycarbonyl-6-O-n-hexyl-3',4'-O-isopropylideneelsamicin A |
| 6g | 2''-N-t-Butoxycarbonyl-6-O-n-decyl-3',4'-O-isopropylideneelsamicin A |
| 6h | 2''-N-t-Butoxycarbonyl-3,4-isopropylidene-6-O-(3-phthalimidopropyl)elsamicin A |
| 7a | 6-O-Methylelsamicin A |
| 7b | 6-O-Ethylelsamicin A |
| 7c | 6-O-n-Propylelsamicin A |
| 7d | 6-O-Isopropylelsamicin A |
| 7e | 6-O-n-Butylelsamicin A |
| 7f | 6-O-n-Hexylelsamicin A |
| 7g | 6-O-n-Decylelsamicin A |
| 7h | 6-O-(3-phthalimidopropyl)elsamicin A |
| 7i | 6-O-(3-Aminopropyl)elsamicin A |
| 8a | 3',4'-O-Isopropylidene-6-O-methylelsamicin A |
| 8b | 6-O-Ethyl-3'4'-O-isopropylideneelsamicin A |
| 8f | 6-O-n-Hexyl-3'4'-O-isopropylideneelsamicin A |
| 8g | 6-O-n-Decyl-3'4'-O-isopropylideneelsamicin A |
| 8h | 3'4'-O-Isopropylidene-6-O-(3-phthalimidopropyl)elsamicin A |
| 8i | 6-O-(3-Aminopropyl)-3',4'-isopropylideneelsamicin A |

**Scheme 1** Synthetic Route of 6-O-Alkyl Derivatives of Elsamicin A

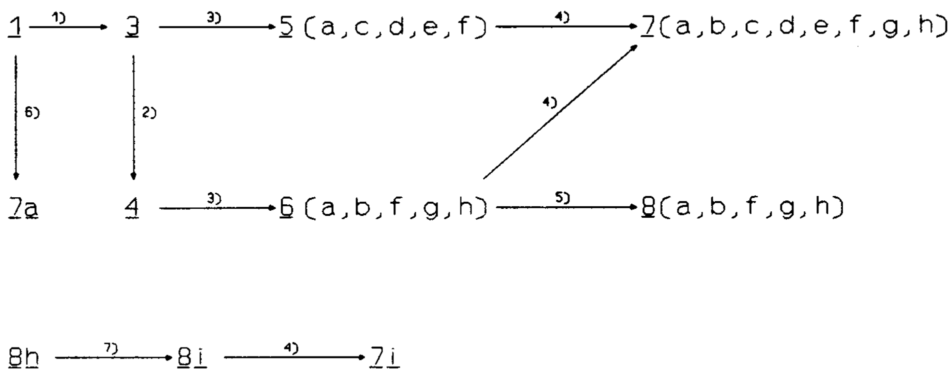


**1)** **$(t\text{-BOC})_2O/NEt_3$**
**2)** **$(CH_3O)_2C(CH_3)_2/TsOH$**
**3)** **Alkyl halide/$K_2CO_3$ in DMSO with or without KI**
**4)** **TFA**
**5)** **TsOH/acetone**
**6)** **$CH_2N_2$**
**7)** **$NH_2NH_2 \bullet H_2O$**

**Scheme 1** (cont'd)

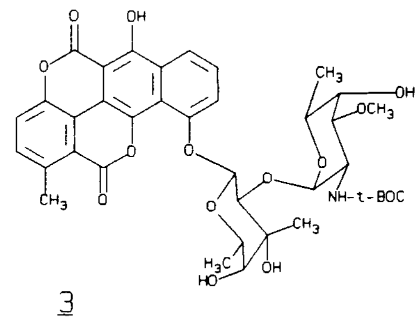

3

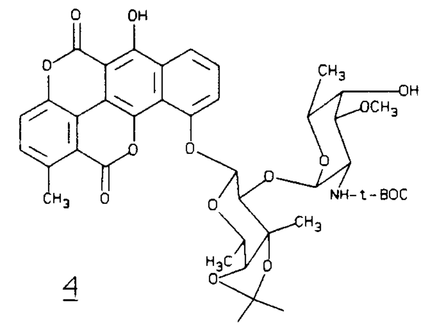

4

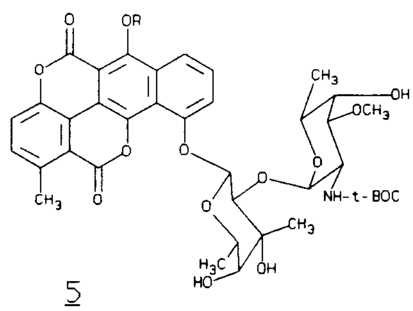

5

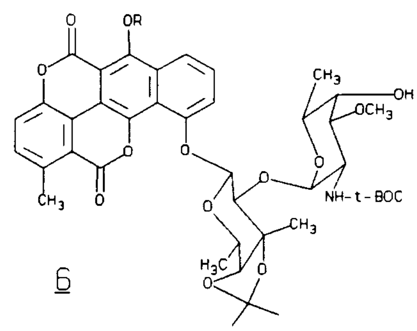

6

**Scheme 1 (cont.)**

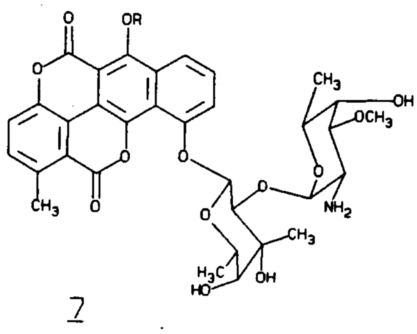

7

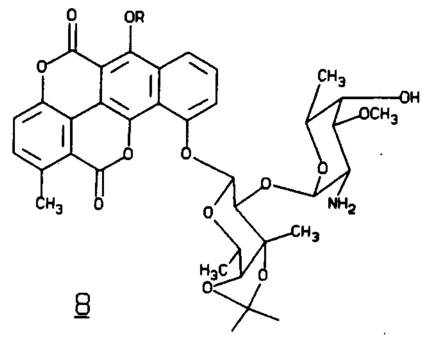

8

| | | | |
|---|---|---|---|
| R | = | (a) | $CH_3$ |
| | | (b) | $C_2H_5$ |
| | | (c) | $n-C_3H_7$ |
| | | (d) | $i-C_3H_7$ |
| | | (e) | $n-C_4H_9$ |
| | | (f) | $n-C_6H_{13}$ |
| | | (g) | $n-C_{10}H_{21}$ |
| | | (h) | 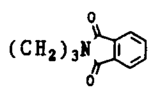 |
| | | (i) | $(CH_2)_3NH_2$ |

Antitumor activity of 6-O-alkylelsamicin A derivatives

Fifteen 6-O-alkylelsamicin A derivatives synthesized and were comparatively tested with the parent compound for in vitro and in vivo antitumor activities.

For in vitro cytotoxicity experiment, murine melanoma B16-F10 cells were grown and maintained in Eagle's minimum essential medium (Nissui), which contains kanamycin (60 μg/ml), supplemented with heat-inactivated fetal calf serum (10%) and nonessential amino acids (0.6%) at 37°C under a humidified atmosphere in a 5% $CO_2$ incubator. Exponentially growing B16-F10 cells were harvested, counted and suspended in the culture medium at the concentration of $2.0 \times 10^4$ cells/ml. The cell suspension (180 μl) was planted into wells of a 96-well microtiter plate and incubated for 24 hours. Test compounds (20 μl) were added to the wells and the plates were further incubated for 72 hours. The cytotoxic activity was colorimetrically determined at 540 nm after staining viable cells with neutral red solution. As shown in Table

2, 6-O-ethylelsamicin A,(7b), 6-O-n-propylelsamicin A, (7c), 6-O-n-butylelsamicin A, (7e) and 6-O-ethyl-3',4'-isopropylideneelsamicin A, (8b) showed approximately equipotent cytotoxicity to the parent compound against B16-F10 cells. The other eleven alkyl derivatives were less active.

In vivo antitumor activity of the above fifteen derivatives was tested in the lymphocytic leukemia P388 and melanoma B16 systems. Female $CDF_1$ (for P388) and male $BDF_1$ (for B16) mice were inoculated by ip injection at $10^6$ P388 cells and 0.5 ml of a 10% B16 brei per mouse, respectively (day 0). Test compounds were intraperitoneally administered to the mice once daily on days 1 to 3 (Q1Dx3) in the P388 system or once a day on days 1, 5 and 9 (Q4Dx3) in the B16 system and animals were observed for 50 days. The percent increase of median survival time (MST) of treated animals over that of untreated control animals was determined and reported as T/C %. Compounds showing T/C % values of 125 or greater are considered to have significant antitumor activity. Similar to the in vitro cytotoxicity, derivatives 7b, 7c, 7e, and 8b were as active as elsamicin A in the P388 system and seemed to be less toxic (Table 2). On the other hand, though 6-O-methylelsamicin A, (7a) and 3',4'-0-isopropylidene-6-O-methylelsamicin A, (8a) were less cytotoxic than the parent compound, they showed approximately equipotent anti-P388 activity in terms of minimum effective doses. As shown in Table 3, seven derivatives tested in the B16 system showed fairly good response to the tumor. In particular, compounds 7a 7b, and 8a demonstrated excellent chemotherapeutic activity with high T/C % values because some survivors were observed at 10 ~ 40 mg/kg/day on day 50.

Table 2. *In vitro* cytotoxicity against B16 melanoma and *in vivo* antitumor activity against P388 leukemia in mice.

| | | Cytotoxicity | T/C % of MST*1 | | | | | | |
|---|---|---|---|---|---|---|---|---|---|
| Compound | R | $IC_{50}$ (μg/ml) | 40*2 | 20 | 10 | 3 | 1 | 0.3 | 0.1 |
| 7a | $CH_3$ | 0.19 | 184 | 205 | 160 | 140 | 125 | 135 | 120 |
| 7b | $C_2H_5$ | 0.06 | 180 | 170 | 170 | 150 | 130 | 125 | 110 |
| 7c | n-$C_3H_7$ | 0.03 | 159 | 155 | 152 | 152 | 133 | 129 | 105 |
| 7d | i-$C_3H_7$ | 0.49 | | | 148 | 133 | 124 | 119 | 95 |
| 7e | n-$C_4H_7$ | 0.03 | 164 | 141 | 162 | 152 | 133 | 124 | 110 |
| 7f | n-$C_6H_{13}$ | 0.11 | | | 140 | 130 | 120 | 105 | 100 |
| 7g | n-$C_{10}H_{21}$ | 2.5 | | | 100 | 115 | 105 | 115 | |
| 7h | $(CH_2)_3$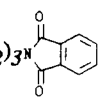 | 0.71 | | | 140 | 125 | 120 | 105 | 105 |
| 7i | $(CH_2)_3NH_2$ | 2.3 | | | 130 | 110 | 110 | 105 | |
| 8a | $CH_3$ | 0.08 | | | 245 | 160 | 135 | 120 | 110 |
| 8b | $C_2H_5$ | 0.06 | | 200 | 190 | 140 | 125 | 125 | 110 |
| 8f | n-$C_6H_{13}$ | 0.9 | | | 120 | 115 | 110 | 110 | 110 |
| 8g | n-$C_{10}H_{21}$ | 2.9 | | | 100 | 115 | 105 | 115 | |
| 8h | $(CH_2)_3$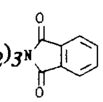 | 0.4 | | | 130 | 120 | 110 | 110 | 100 |
| 8i | $(CH_2)_3NH_2$ | 1.8 | | | 135 | 120 | 110 | 105 | |
| Elsamicin A (1) | | 0.05 | | Tox | 206 | 180 | 155 | 138 | 124 |

*1 Median survival time in days

*2 Dose in mg/kg/day, Q1Dx3ip

Table 3

| In vivo antitumor activity against B16 melanoma in mice | | | | | | | |
|---|---|---|---|---|---|---|---|
| Compound | T/C % of MST*1 | | | | | | |
| | 40*2 | 20 | 10 | 3 | 1 | 0.3 | 0.1 |
| 7a | ≧370 (3/4)*3 | ≧370 (3/4) | 262 | 197 | 147 | 121 | 106 |
| 7b | | | ≧313 (3/4) | 178 | 144 | 113 | 103 |
| 7e | | | 213 | 160 | 127 | 123 | |
| 7f | | | 146 | 132 | 129 | 111 | 107 |
| 7g | | | 183 | 153 | 126 | 107 | |
| 8a | | ≧370 (3/4) | 285 | 215 | 141 | 104 | |
| 8b | | | 216 | 153 | 119 | 106 | 97 |
| Elsamicin A (1) | | | ≧305 (9/24) | ≧216 (2/24) | 178 | 139 | 120 |

*1 Median survival times in days
*2 Dose in mg/kg/day, Q4Dx3 ip
*3 No. of survivors/tested on day 50

The present invention includes within its scope a process for producing the elsamicin A derivatives of the present invention.

Another aspect of the invention, there are provided pharmaceutical compositions which comprise an effective tumor-inhibiting amount of the compound of Formula III or IV, in combination with an inert pharmaceutically acceptable carrier or diluent.

According to another aspect of the invention provides a method for therapeutically treating an animal, preferably mammalian, host affected by a tumor which comprises administering to such host an effective tumor-inhibiting dose of the antibiotic of the compound of Formula III or IV.

Examples of suitable compositions include solid compositions for oral administration such as tablets, capsules, pills, powders and granules, liquid compositions for oral administration such as solutions, suspensions, syrups and elixirs and preparations for parenteral administration such as sterile solutions, suspensions or emulsions. They may also be manufactured in the form of sterile solid compositions which can be dissolved in sterile water, physiological saline or some other sterile injectable medium immediately before use.

It will be appreciated that the actual preferred dosages of the elsamicin A derivative of the present invention will vary according to the particular compound being used, the particular composition formulated, the mode of application and the particular situs, host and disease being treated. Many factors that modify the action of the drug will be taken into account by those skilled in the art, e.g. age, body weight, sex, diet, time of administration, rate of excretion, condition of the host, drug combinations, reaction sensitivities and severity of the disease. Administration can be carried out continuously or periodically within the maximum tolerated dose. Optimal application rates for a given set of conditions can be readily ascertained by those skilled in the art using conventional dosage determination tests.

The present invention is illustrated by the following examples which are not intended to be construed as limiting the scope of the invention.

Specific synthesis examples of the derivatives 3 - 6h are explained below, from which derivatives the compounds of this invention 7 and 8 are synthesized by the above process.

EXAMPLE 1

Synthesis of 2''-N-t-Butoxycarbonylelsamicin A (3)

A mixture of elsamicin A (653 mg, 1 mmole), di-t-butyl dicarbonate (348 mg, 1.6 mmoles), and triethylamine (0.14 ml, 1 mmole) in dioxane (10 ml) was stirred at room temperature overnight. The reaction mixture was evaporated in vacuo to give semi-crystalline residue. The residue was recrystallized from $CH_2Cl_2$/ether to obtain 768 mg (100%) of yellow crystalline powder.

MP 183-184 °C. IR $\nu_{max}$ (KBr) $cm^{-1}$ 3410, 1700, 1505, 1370, 1255, 1120, 1065, 875, 780. UV $\lambda_{max}$ - (MeOH) nm ($\epsilon$) 236 (38300), 266 (37800), 333 (6180), 380 (8770), 400 (14500), 423 (15900). $^1$H NMR

($CDCl_3$) δ 0.73 (9H, br.s.), 1.31 (3H, d, J=7 Hz), 1.36 (3H, s), 1.39 (3H, d, J=6 Hz), 2.68 (3H, s), 3.35 (3H, s), 5.37 (1H, d, J=8 Hz), 4.66 (1H, d, J=4 Hz), 8.18 (1H, dd, J=8 & 1.5 Hz), 11.59 (1H, s).

| Anal Calcd for $C_{38}H_{43}NO_{15} \cdot H_2O$: | | | |
|---|---|---|---|
| | C 59.14, | H 5.88, | N 1.81. |
| Found: | C 59.12, | H 6.06, | N 2.27. |

EXAMPLE 2

Synthesis of 2''-N-t-Butoxycarbonyl-3'-,4'-0-isopropylideneelsamicin A (4)

To a solution of 2''-N-t-butoxycarbonylelsamicin A (3), (200 mg) and 2,2-dimethoxypropane (1.2 ml) in dry $CH_2Cl_2$ (4 ml) was added TsOH (5 mg), and the mixture was stood at room temperature overnight. A saturated aqueous $NaHCO_3$ was added, and the reaction mixture was vigorously stirred. The organic layer was taken up, dried with $MgSO_4$, and evaporated in vacuo to give 190 mg (90%) of yellow solid.
MP 168-169°C. IR $\nu_{max}$ (KBr) $cm^{-1}$ 3420, 1740, 1690, 1505, 1375, 1250, 1065, 780. UV $\lambda_{max}$ (MeOH) nm (ε) 236 (40600), 266 (39100), 333 (6370), 380 (8850), 399 (14200), 422 (15500). $^1H$ NMR ($CDCl_3$) δ 1.34 (3H, d, J=6 Hz), 1.37 (3H, d, J=7 Hz), 1.37 (3H, s), 1.42 (3H, s), 1.68 (3H, s), 2.87 (3H, s), 3.35 (3H, s), 5.23 (1H, d, J=8 Hz), 5.80 (1H, d, J=4 Hz), 8.33 (1H, dd, J=8 & 1.5 Hz), 1.63 (1H, br.).

| Anal Calcd for $C_{41}H_{47}NO_{15} \cdot H_2O$: | | | |
|---|---|---|---|
| | C 60.66, | H 6.08, | N 1.73. |
| Found: | C 60.92, | H 6.03, | N 2.00. |

EXAMPLE 3

Synthesis of 2''-N-t-Butoxycarbonyl-6-0-methylelsamicin A (5a)

A mixture of 2''-N-t-butoxycarbonylelsamicin A (3), (753 mg), methyliodide (186 µl), and $K_2CO_3$ (276 mg) in dry DMSO (10 ml) was stirred at room temperature for 24 hrs. The reaction mixture was diluted with water and extracted with $CHCl_3$. The extract was dried with $MgSO_4$ and evaporated under reduced pressure to give an oily residue. The residue was purified by silica gel column chromatography using 1-3% MeOH in $CHCl_3$ as eluants to obtain 572 mg (75%) of the title product.
MP 183-185°C. IR $\nu_{max}$ (KBr) $cm^{-1}$ 1740, 1610, 1495, 1365, 1250, 1070, 1050. UV $\lambda_{max}$ (MeOH) nm (ε) 236 (36000), 266 (40300), 327 (7070), 369 (8830), 390 (12700), 411 (13700). $^1H$ NMR ($CDCl_3$) δ 0.77 (9H, s), 1.27 (3H, d, J=6 Hz), 1.38 (3H, s), 1.42 (3H, d, J=6 Hz), 2.85 (3H, s), 3.38 (3H, s), 4.15 (3H, s), 5.39 (1H, d, J=8 Hz), 5.63 (1H, d, J=4 Hz).

| Anal Calcd for $C_{39}H_{45}NO_{15} \cdot H_2O$: | | | |
|---|---|---|---|
| | C 59.61, | H 6.03, | N 1.78. |
| Found: | C 59.54, | H 5.76, | N 1.64. |

Derivatives 5c - 5f were prepared from intermediate 3 by a similar method to that described in the preparation of intermediate 5a. Alkylating agents, isolation yields and physicochemical data of the products are given.

EXAMPLE 4

Synthesis of 2''-N-t-Butoxycarbonyl-6-0-n-propylelsamicin A (5c)

Alkylation reagent: n-propyl iodide Yield: 77%
MP 167-169°C. IR $\nu_{max}$ (KBr) $cm^{-1}$ 3420, 1735, 1360, 1255, 1070. UV $\lambda_{max}$ (MeOH) nm (ε) 237 (35400), 266

(39400), 328 (7190), 371 (8920), 391 (12900), 411 (13900). $^1H$ NMR ($CDCl_3$) δ 0.77 (9H, s), 1.05-1.50 (12H, m), 2.08 (3H, s), 3.38 (3H, s), 5.40 (1H, d, J=8 Hz), 5.68 (1H, d, J=4 Hz).

| Anal Calcd for $C_{41}H_{49}NO_{15}\cdot H_2O$: | | | |
|---|---|---|---|
| | C 60.51, | H 6.32, | N 1.72. |
| Found: | C 60.62, | H 6.23, | N 1.66. |

EXAMPLE 5

Synthesis of 2''-N-t-Butoxycarbonyl-6-0-isopropylelsamicin A (5d)

Alkylation reagent: isopropyl iodide Yield: 81%
MP 169-171 °C. IR $\nu_{max}$ (KBr) $cm^{-1}$ 3420, 1730, 1370, 1250, 1075. UV $\lambda_{max}$ (MeOH) nm (ε) 236 (35700), 266 (39200), 329 (7040), 372 (8760), 393 (12800), 414 (13800). $^1H$ NMR ($CDCl_3$) δ 0.80 (9H, s), 1.15-2.00 (15H, m), 2.85 (3H, s), 3.88 (3H, s), 4.22 (1H, m), 5.38 (1H, d, J=8 Hz), 5.60 (1H, d, J=4 Hz).

| Anal Calcd for $C_{41}H_{49}NO_{15}\cdot 1/2H_2O$: | | | |
|---|---|---|---|
| | C 61.19, | H 6.26, | N 1.74. |
| Found: | C 61.06, | H 6.15, | N 1.70. |

EXAMPLE 6

Synthesis of 2''-N-t-Butoxycarbonyl-6-0-n-butylelsamicin A (5e)

Alkylation reagent: n-butyl iodide Yield: 81%
MP 164-166 °C. IR $\nu_{max}$ (KBr) $cm^{-1}$ 3420, 1725, 1360, 1255, 1070. UV $\lambda_{max}$ (MeOH) nm (ε) 237 (36600), 265 (40400), 327 (7460), 371 (9230), 391 (13300), 411 (14300). $^1H$ NMR ($CDCl_3$) δ 0.78 (9H, s), 1.05 (3H, t, J=7 Hz), 1.32 (3H, d, J=6 Hz), 1.38 (3H, s), 1.42 (3H, d, J=6 Hz), 1.61 (2H, m), 1.98 (2H, m), 2.84 (3H, s), 3.38 (3H, s), 5.40 (1H, d, J=8 Hz), 5.63 (1H, d, J=4 Hz).

| Anal Calcd for $C_{42}H_{51}NO_{15}\cdot H_2O$: | | | |
|---|---|---|---|
| | C 60.93, | H 6.45, | N 1.69. |
| Found: | C 61.08, | H 6.37, | N 1.76. |

EXAMPLE 7

Synthesis of 2''-N-t-Butoxycarbonyl-6-0-n-hexylelsamicin A (5f)

Alkylation reagent: n-hexyl bromide (Alkylation reaction was carried out in the presence of KI) Yield: 84%
MP 145-147 °C. IR $\nu_{max}$ (KBr) $cm^{-1}$ 1740, 1360, 1250, 1165, 1105, 1070, 780. UV $\lambda_{max}$ (MeOH) nm (ε) 237 (37700), 266 (41000), 327 (7690), 370 (9260), 391 (13300), 412 (14300). $^1H$ NMR ($CDCl_3$) δ 0.75 (9H, s), 0.94 (3H, t, J=6 Hz), 1.27 (3H, d, J=6 Hz), 1.34 (3H, s), 1.42 (3H, d, J=6 Hz), 2.83 (3H, s), 3.37 (3H, s), 5.38 (1H, d, J=8 Hz), 5.61 (1H, d, J=4 Hz), 8.20 (1H, d, J=8 Hz).

| Anal Calcd for $C_{44}H_{55}NO_{15}\cdot H_2O$: | | | |
|---|---|---|---|
| | C 61.74, | H 6.71, | N 1.64. |
| Found: | C 61.88, | H 6.58, | N 1.57. |

EXAMPLE 8

Synthesis of 2''-N-t-Butoxycarbonyl-3',4'-0-isopropylidene-6-0-methylelsamicin A (6a)

A mixture of 2''-N-t-butoxycarbonyl-3',4'-0-isopropylideneelsamicin A (4), (952 mg), $K_2CO_3$ (995 mg), and MeI (1.02 g) in DMSO (20 ml) was stirred at room temperature for 18 hrs and at 60 °C for 2 hours. The reaction mixture was diluted with water and extracted with EtOAc. The extract was washed with water, dried over $Na_2SO_4$, and evaporated in vacuo. The residual oil was purified by column chromatography on silica gel using $CH_3OH$ in $CH_2Cl_2$ (0%-2%) as eluants to obtain 831 mg (86%) of the title compound.
MP 160 ° -162 ° C. IR $\nu_{max}$ (KBr) $cm^{-1}$ 3450, 2980, 2860, 1745, 1715sh. UV $\lambda_{max}$ (MeOH) nm ($\epsilon$) 236 (35400), 266 (39600), 327 (6960), 366 (8630), 390 (12100), 410 (12800). $^1$H NMR ($CDCl_3$) $\delta$ 0.65 (9H, s), 1.38 (9H, m), 1.65 (3H, s), 1.70 (3H, s), 2.82 (3H, s), 3.38 (3H, s), 4.15 (3H, s), 5.24 (1H, d, J = 8 Hz), 5.82 (1H, d, J = 4 Hz), 8.21 (1H, d, J = 8 Hz).

| Anal Calcd for $C_{42}H_{49}NO_{15}$ • 1.5$H_2O$: | | | |
|---|---|---|---|
| | C 60.42, | H 6.28, | N 1.68. |
| Found: | C 60.16, | H 5.96, | N 1.61. |

Intermediates 6b, 6f, 6g, and 6h were prepared from intermediate 4 by a similar method to that described in the preparation of intermediate 6a. Alkylation reagents, isolation yields and physicochemical data are given.

EXAMPLE 9

Synthesis of 2''-N-t-Butoxycarbonyl-6-0-ethyl-3',4'-0-isopropylideneelsamicin A (6b)

Alkylation reagent: Ethyl bromide (the reaction was carried out in the presence of KI) Yield: 94%
MP 161-183 ° C. IR $\nu_{max}$ (KBr) $cm^{-1}$ 3420, 2970, 2925, 2860, 1735. UV $\lambda_{max}$ (MeOH) nm ($\epsilon$) 236 (35800), 266 (40100), 326 (7360), 369 (8960), 390 (12700), 411 (13200). $^1$H NMR ($CDCl_3$) $\delta$ 0.65 (9H, s), 1.15-1.85 (18H), 2.90 (3H, s), 4.25 (2H, q, J = 7 Hz), 5.25 (1H, d, J = 8 Hz), 5.80 (1H, d, J = 4 Hz), 8.23 (1H, dd, J = 8 & 1 Hz).

| Anal. Calcd. for $C_{43}H_{51}NO_{15}$ • $H_2O$: | | | |
|---|---|---|---|
| | C 61.49, | H 6.36, | N 1.67. |
| Found: | C 61.67, | H 6.19, | N 1.66. |

EXAMPLE 10

Synthesis of 2''-N-t-Butoxycarbonyl-6-0-n-hexyl-3',4'-0-isopropylideneelsamicin A (6f)

Alkylation reagent: n-Hexyl bromide (in the presence of KI) Yield: 98%
MP 143-145 ° C. IR $\nu_{max}$ (KBr) $cm^{-1}$ 3440, 2970, 2930, 2855, 1740. UV $\lambda_{max}$ (MeOH) nm ($\epsilon$) 236 (37900), 266 (40800), 327 (7450), 368 (8960), 391 (12600), 411 (13400). $^1$H NMR ($CDCl_3$) $\delta$ 0.65 (9H, s), 0.93 (3H, t, J = 7Hz), 1.38 (15H, m), 1.63 (3H, s), 1.70 (3H, s), 2.05 (2H, m), 2.90 (3H, s), 3.38 (3H, s), 4.17 (2H, q, J = 7 Hz), 5.24 (1H, d, J = 8 Hz), 5.79 (1H, d, J = 4 Hz).

| Anal. Calcd. for $C_{47}H_{59}NO_{15}$ • $H_2O$: | | | |
|---|---|---|---|
| | C 63.00, | H 6.86, | N 1.56. |
| Found: | C 63.12, | H 7.01, | N 1.52. |

EXAMPLE 11

Synthesis of 2''-N-t-Butoxycarbonyl-6-0-n-decyl-3',4'-0-isopropylideneelsamicin A (6g)

Alkylation reagent: n-Decyl bromide (in the presence of KI) Yield: 50%
MP 113-115°C. IR $\nu_{max}$ (KBr) $cm^{-1}$ 3440, 2970, 2920, 2840, 1740. UV $\lambda_{max}$ (MeOH) nm ($\epsilon$) 236 (38100), 266 (41600), 327 (8200), 367 (9530), 390 (13300), 411 (14000). $^1H$ NMR ($CDCl_3$) $\delta$ 0.65 (9H, s), 0.89 (3H, m), 1.05-1.80 (29H, m), 2.05 (2H, m), 2.90 (3H, s), 3.39 (3H, s), 4.16 (2H, t, J=7 Hz), 5.25 (1H, d, J=8 Hz), 5.80 (1H, d, J=4 Hz).

| Anal. Calcd. for $C_{51}H_{67}NO_{15}\bullet 1/2H_2O$: | | | |
|---|---|---|---|
| | C 64.95, | H 7.27, | N 1.49. |
| Found: | C 64.98, | H 7.24, | N 1.46. |

EXAMPLE 12

Synthesis of 2''-N-t-Butoxycarbonyl-3',4'-O-isopropylidene-6-0-(3-phthalimidopropyl)elsamicin A (6h)

Alkylation reagent: 3-Phthalimido-propyl chloride (in the presence of KI) Yield = 100%
MP 157-159°C. IR $\nu_{max}$ (KBr) $cm^{-1}$ 3340, 1740, 1710, 1495, 1395, 1365, 1250, 1070, 785. UV $\lambda_{max}$ (MeOH) nm ($\epsilon$) 220 (55900), 234 (49000), 267 (40100), 326 (7560), 368 (8930), 391 (12500), 411 (13100). $^1H$ NMR ($CDCl_3$) $\delta$ 0.70 (9H, s), 1.25-1.5 (12H, m), 1.70 (3H, s), 2.43 (2H, quint, J=7 Hz), 2.90 (3H, s), 3.38 (3H, s), 5.25 (1H, d, J=8 Hz), 5.82 (1H, d, J=4 Hz).

| Anal. Calcd. for $C_{52}H_{56}N_2O_{17}\bullet 1/2H_2O$: | | | |
|---|---|---|---|
| | C 63.33, | H 5.55, | N 2.69. |
| Found: | C 63.32, | H 5.68, | N 2.77. |

EXAMPLE 13

Synthesis of 6-0-Methylelsamicin A (7a)

(Method A)

An etheral solution of diazomethane (prepared from 500 mg of N-nitrosomethylurea) was added to a suspension of elsamicin A (130 mg) in dioxane (20 ml). The reaction mixture was stirred at room temperature for 3 hours, treated with the same amount of etheral diazomethane, stirred again at room temperature for additional 1 hour and evaporated in vacuo to give an amorphous yellow solid. The solid was purified by successive preparative TLCs using $CHCl_3$-MeOH-28% $NH_4OH$ (8:3:1) and $CHCl_3$-MeOH (4:1) as developing solvents to give 73 mg (55%) of the title compound.
MP 176-179°C. IR $\nu_{max}$ (KBr) $cm^{-1}$ 3400, 1735, 1365, 1250, 1105, 1075, 780. UV $\lambda_{max}$ (MeOH) nm ($\epsilon$) 237 (37600), 265 (41600), 327 (7680), 368 (9140), 390 (13100), 410 (13900). $^1H$ NMR ($CDCl_3$ + $D_2O$) $\delta$ 1.30 (3H, d, J=6.0 Hz), 1.38 (3H, s), 1.40 (3H, d, J=6 Hz), 2.82 (3H, s), 3.39 (3H, s), 4.14 (3H, s), 5.35 (1H, d, J=8 Hz), 5.54 (1H, d, J=4 Hz), 8.12 (1H, d J=8 Hz).

| Anal. Calcd. for $C_{35}H_{37}NO_{13}\bullet 1/2H_2O$: | | | |
|---|---|---|---|
| | C 60.35, | H 5.66, | N 2.07 |
| Found: | C 60.05, | H 5.68, | N 2.57. |

SIMS M/Z 668 $(M + H)^+$, 348, 320, 160.

(Method B)

2''-N-t-Butoxycarbonyl-6-0-methylelsamicin (5a), 321 mg) was dissolved in 90% trifluoroacetic acid (1 ml). The reaction mixture was kept at room temperature for 5 minutes and concentrated under reduced pressure. To the residue was added a mixture of saturated aqueous $NaHCO_3$ (10 ml) and $CHCl_3$. The $CHCl_3$ layer was taken up, dried with $Na_2SO_4$ and evaporated to give a yellow residue, which was purified

by silica gel column chromatography to obtain 217 mg (78%) of the desired product. MP 176-178°C. Spectral data of this product were completely identical with those of the product from elsamicin A and diazomethane.

(Method C)

2''-N-t-Butoxycarbonyl-3',4'-0-isopropylidene-6-0-methylelsamicin A (6a), (242 mg) was dissolved in trifluoroacetic acid (0.5 ml) at room temperature. After 5 minutes, trifluoroacetic acid was evaporated under reduced pressure and the residue was dissolved in a mixture of a saturated aqueous $NaHCO_3$ (10 ml) and $CHCl_3$ (10 ml). The organic layer was taken up, dried with $Na_2SO_4$ and evaporated to give a yellow mass, which was purified by silica gel column chromatography to obtain 154 mg (77%) of the desired product. MP 176-178°C. Spectral data of this product were completely identical with those obtained in Method A.

Compounds 7c - 7f were prepared from derivatives 5c-5f, respectively, by a similar procedure to that described in the preparation of compounds 7a from derivative 5a (Method B). Isolation yields and physicochemical data of the product are given.

EXAMPLE 14

Synthesis of 6-0-n-Propylelsamicin A (7c)

Yield from 5c: 72%
MP 159-161°C. IR $\nu_{max}$ (KBr) $cm^{-1}$ 3420, 1735, 1360, 12505, 1110. UV $\lambda_{max}$ (MeOH) nm ($\epsilon$) 237 (34900), 266 (38600), 327 (7310), 368 (8560), 396 (12300), 411 (13000).
$^1H$ NMR ($CDCl_3$) $\delta$ 1.15 (3H, t, J=7.5 Hz), 1.30 (3H, d, J=7 Hz), 1.38 (3H, s), 1.39 (3H, d, J=7 Hz), 2.06 (2H, m), 2.85 (3H, s), 3.40 (3H, s), 4.14 (2H, t, J=7.5 Hz), 5.37 (1H, d, J=8 Hs), 5.55 (1H, d, J=4 Hz).

| Anal. Calcd. for $C_{36}H_{41}NO_{13}\cdot 3/2H_2O$: | | | |
|---|---|---|---|
| | C 59.83, | H 6.14, | N 1.94. |
| Found: | C 59.99, | H 5.92, | N 1.88. |

EXAMPLE 15

Synthesis of 6-0-Isopropylelsamicin A (7d)

Yield from 5d: 81%
MP 167-169°C. IR $\nu_{max}$ (KBr) $cm^{-1}$ 3400, 1735, 1370, 1250, 1080. UV $\lambda_{max}$ (MeOH) nm ($\epsilon$) 236 (36400), 266 (40000), 328 (7450), 371 (8910), 392 (12900), 414 (13600). $^1H$ NMR ($CDCl_3$) $\delta$ 1.35 (15H, m), 2.85 (3H, s), 3.39 (3H, s), 5.35 (1H, d, J=8 Hz), 5.55 (1H, d, J=4 Hz).

| Anal. Calcd. for $C_{36}H_{41}NO_{13}\cdot 1.5H_2O$: | | | |
|---|---|---|---|
| | C 59.83, | H 6.14, | N 1.94. |
| Found: | C 59.90, | H 5.90, | N 1.81. |

EXAMPLE 16

Synthesis of 6-0-n-Butylelsamicin A (7e)

Yield from 5e: 84%
MP 153-155°C. IR $\nu_{max}$ (KBr) $cm^{-1}$ 3380, 1735, 1355, 1250, 1110, 1075. UV $\lambda_{max}$ (MeOH) nm ($\epsilon$) 237 (37300), 266 (41300), 327 (7880), 369 (9160), 390 (13200), 411 (14100). $^1H$ NMR ($CDCl_3$) $\delta$ 1.04 (3H, t, J=7 Hz), 1.30 (3H, d, J=7 Hz), 1.38 (3H, d, J=7 Hz), 1.38 (3H, s), 1.8 (4H, m), 2.85 (3H, s), 3.38 (3H, s), 5.35 (1H, d, J=8 Hz), 5.55 (1H, d, J=4 Hz).

| Anal. Calcd. for $C_{37}H_{43}NO_{13}\cdot 1.5H_2O$: | | | |
|---|---|---|---|
| | C 60.32, | H 6.29, | N 1.90. |
| Found: | C 60.43, | H 6.06, | N 1.88. |

EXAMPLE 17

Synthesis of 6-0-n-Hexylelsamicin A (7f)

Yield from 5f: 89%
MP 133-135°C. IR $\nu_{max}$ (KBr) $cm^{-1}$ 1735, 1355, 1250, 1105, 1070, 780. UV $\lambda_{max}$ (MeOH) nm ($\epsilon$) 237 (38600), 266 (42300), 326 (8140), 369 (9380), 390 (13400), 410 (14100).
$^1H$ NMR ($CDCl_3$) $\delta$ 0.93 (3H, t, J=6 Hz), 1.28 (3H, d, J=6 Hz), 1.38 (3H, s), 1.39 (3H, d, J=7 Hz), 2.83 (3H, s), 3.39 (3H, s), 5.34 (1H, d, J=7 Hz), 5.54 (1H, d, J=4 Hz), 8.16 (1H, dd, J=8 & 1 Hz).

| Anal. Calcd. for $C_{39}H_{47}NO_{13}\cdot 1/2H_2O$: | | | |
|---|---|---|---|
| | C 62.72, | H 6.48, | N 1.88. |
| Found: | C 62.54, | H 6.35, | N 1.79. |

SIMS M/Z: 738 $(M + H)^+$, 654, 419, 320, 160.

Compounds 7b, 7g and 7h also were prepared from derivatives 6b, 6g and 6h, respectively, by a similar method to that described in the preparation of compound 7a from derivative 6a (Method C). Isolation yields and physicochemical data of the products are given.

EXAMPLE 18

Synthesis of 6-0-Ethylelsamicin A (7b)

Yield from 6b: 88%
MP 164-166°C. IR $\nu_{max}$ (KBr) $cm^{-1}$ 3420, 2970, 2920, 2860, 1740. UV $\lambda_{max}$ (MeOH) nm ($\epsilon$) 237 (36200), 265 (40200), 327 (7500), 368 (8870), 390 (12800), 411 (13500). $^1H$ NMR ($CDCl_3$) $\delta$ 1.30 (3H, d, J=7.5 Hz), 1.38 (3H, s), 1.38 (3H, d, J=7.5 Hz), 1.62 (3H, t, J=7 Hz), 2.85 (3H, s), 3.40 (3H, s), 4.26 (2H, q, J=7 Hz), 5.35 (1H, d, J=8 Hz), 5.55 (1H, d, J=4 Hz), 8.16 (1H, dd, J=8 & 1.5 Hz).

| Anal. Calcd. for $C_{35}H_{39}NO_{13}\cdot H_2O$: | | | |
|---|---|---|---|
| | C 60.48, | H 5.91, | N 2.00. |
| Found: | C 59.85, | H 5.78, | N 1.85. |

EXAMPLE 19

Synthesis of 6-0-n-Decylelsamicin A (7g)

Yield from 6g: 66%
MP 116-118°C. IR $\nu_{max}$ (KBr) $cm^{-1}$ 3420, 2920, 2840, 1740. UV $\lambda_{max}$ (MeOH) nm ($\epsilon$) 237 (36300), 266 (40100), 327 (7690), 369 (8970), 390 (12800), 411 (13600). $^1H$ NMR ($CDCl_3$) $\delta$ 0.89 (3H, m), 1.35 (23H, m), 2.0 (2H, m), 2.87 (3H, s), 3.40 (3H, s), 4.17 (2H, t, J=7 Hz), 5.35 (1H, d, J=8 Hz), 5.55 (1H, d, J=4 Hz).

| Anal. Calcd. for $C_{43}H_{55}NO_{13}\cdot 1/2H_2O$: | | | |
|---|---|---|---|
| | C 64.32, | H 7.03, | N 1.74. |
| Found: | C 64.47, | H 7.09, | N 1.69. |

EXAMPLE 20

Synthesis of 6-0-(3-phthalimidopropyl)elsamicin A (7h)

Yield from 6h: 78%
MP 165-168 °C. IR $\nu_{max}$ (KBr) $cm^{-1}$ 3400, 1740, 1715, 1400, 1360, 1255, 1110, 1075, 1050, 785. UV $\lambda_{max}$ (MeOH) nm ($\epsilon$) 220 (54800), 233 (46700), 266 (38500), 326 (7590), 368 (8600), 390 (12200), 411 (12800). $^1H$ NMR ($CDCl_3$ + $D_2O$) $\delta$ 1.27 (3H, d, J = 6 Hz), 1.36 (3H, s), 1.37 (3H, d, J = 6 Hz), 2.4 (2H, m), 2.80 (3H, s), 3.38 (3H, s), 5.33 (1H, d, J = 8 Hz), 5.52 (1H, d, J = 4 Hz).

EXAMPLE 21

Synthesis of 6-0-(3-Aminopropyl)elsamicin A (7i)

6-0-(3-Aminopropyl)-3',4'-0-isopropylideneelsamicin A (8i), (53 mg) was dissolved in 90% aqueous TFA (0.5 ml) by the aid of sonication. The solution was evaporated under reduced pressure. The residue was dissolved in an aqueous $NaHCO_3$ solution (ca. 5 ml) and the solution was subjected to column chromatography on HP-20 (10x140 mm). The fractions containing the desired product were combined and evaporated to give 39 mg (78%) of compound 7i.
MP 197-206 °C (dec.). IR $\nu_{max}$ (KBr) $cm^{-1}$ 1735, 1680, 1360, 1255, 1110, 1075, 1045, 780. UV $\lambda_{max}$ (MeOH) nm ($\epsilon$) 236 (32200), 267 (33700), 312 (5590), 326 (6490), 369 (7400), 391 (10100), 412 (10400). $^1H$ NMR ($CDCl_3$ + $CD_3OD$) $\delta$ 1.2-1.5 (9H, m), 2.22 (2H, m), 2.88 (3H, s), 3.34 (3H, s), 5.40 (1H, d, J = 8 Hz), 5.58 (1H, d, J = 4 Hz).
SIMS M/Z: 711 $(M + H)^+$, 392, 334, 160.

EXAMPLE 22

Synthesis of 3',4'-0-Isopropylidene-6-0-methylelsamicin A (8a)

A solution of 2''-N-t-butoxycarbonyl-3',4'-0-isopropylidene-6-0-methylelsamicin A (6a), (100 mg) and p-toluenesulfonic acid monohydrate (118 mg) in acetone (2 ml) was stirred at room temperature for 2 hours and evaporated under reduced pressure. The oily residue was dissolved in a mixture of MeOH and $CHCl_3$ (1:9) and the solution washed with aqueous 10% $NaHCO_3$, water and brine, successively, dried over $Na_2SO_4$ and evaporated in vacuo to give a yellowish sticky solid. The solid was purified by preparative TLC to give 61 mg (69%) of the title compound.
MP 168-170 °C. IR $\nu_{max}$ (KBr) $cm^{-1}$ 3420, 2980, 2925, 2860, 1740. UV $\lambda_{max}$ (MeOH) nm ($\epsilon$) 237 (36000), 266 (40600), 325 (7360), 367 (8780), 389 (12200), 409 (12600). $^1H$ NMR ($CDCl_3$) $\delta$ 1.39(12H, m), 1.70 (3H, s), 2.88 (3H, s), 3.38 (3H, s), 4.14 (3H, s), 5.24 (1H, d, J = 8 Hz), 5.80 (1H, d, J = 4 Hz), 8.16 (1H, dd, J = 8 &, 1.5 Hz).

| Anal. Calcd. for $C_{37}H_{41}NO_{13} \cdot 5/2H_2O$: | | | |
|---|---|---|---|
| | C 59.04, | H 6.16, | N 1.86. |
| Found: | C 58.87, | H 5.64, | N 2.01. |

Compounds 8b, 8f, 8g and 8h were prepared from derivatives 6b, 6f, 6g and 6h, respectively, by a similar method to that described in the preparation of compound 8a from derivative 6a. Isolation yields and physicochemical data of the products are given.

EXAMPLE 23

Synthesis of 6-O-Ethyl-3',4'-0-isopropylideneelsamicin A (8b)

Yield from 6b: 76%
MP 156-158 °C. IR $\nu_{max}$ (KBr) $cm^{-1}$ 3375, 2980, 2930, 2860, 1740. UV $\lambda_{max}$ (MeOH) nm ($\epsilon$) 236 (35600), 266 (39700), 326 (7440), 368 (8660), 389 (12100), 409 (12600). $^1H$ NMR ($CDCl_3$) $\delta$ 1.1-1.8 (18H, m), 2.88 (3H, s), 3.38 (3H, s), 4.25 (2H, q, J = 7 Hz), 5.23 (1H, d, J = 8 Hz), 5.80 (1H, d, J = 4 Hz), 8.17 (1H, d, J = 8 Hz).

| Anal Calcd for $C_{38}H_{43}NO_{13} \bullet 3/2H_2O$: | | | |
|---|---|---|---|
| | C 60.95, | H 6.19, | N 1.87. |
| Found: | C 61.14, | H 6.01, | N 1.96. |

EXAMPLE 24

Synthesis of 6-0-n-Hexyl-3',4'-0-isopropylideneelsamicin A (8f)

Yield from 6f: 76%
MP 132-134 °C. IR $\nu_{max}$ (KBr) $cm^{-1}$ 3420, 2970, 2920, 2850, 1740. UV $\lambda_{max}$ (MeOH) nm ($\epsilon$) 236 (35900), 266 (40100), 326 (7640), 367 (8870), 389 (12400), 410 (12800). $^1$H NMR ($CDCl_3$) $\delta$ 0.95 (3H, m), 1.15-1.80 (21H, m), 2.04 (2H, m), 2.83 (3H, s), 3.38 (3H, s), 4.17 (2H, t, J = 7 Hz), 5.22 (1H, d, J = 8 Hz), 5.73 (1H, d, J = 4 Hz).

| Anal calcd for $C_{42}H_{51}NO_{13} \bullet 1/2H_2O$: | | | |
|---|---|---|---|
| | C 64.11, | H 6.66, | N 1.78. |
| Found: | C 63.89, | H 6.61, | N 1.72. |

EXAMPLE 25

Synthesis of 6-O-n-Decyl-3',4'-O-isopropylideneelsamicin A (8g)

Yield from 6g: 84%
MP 101-102 °C. IR $\nu_{max}$ (KBr) $cm^{-1}$ 3380, 2920, 2840, 1740. UV $\lambda_{max}$ (MeOH) nm ($\epsilon$) 236 (36300), 266 (40400), 326 (7750), 368 (9010), 390 (12500), 411 (13000). $^1$H NMR ($CDCl_3$) $\delta$ 0.88 (3H, m), 1.1-1.8 (29H, m), 2.03 (2H, m), 2.87 (3H, s), 3.37 (3H, s), 4.15 (2H, t, J = 7 Hz), 5.21 (1H, d, J = 8 Hz), 5.78 (1H, d, J = 4 Hz).

| Anal Calcd for $C_{46}H_{59}NO_{13} \bullet 1/2H_2O$: | | | |
|---|---|---|---|
| | C 65.54, | H 7.17, | N 1.66. |
| Found: | C 65.30, | H 7.29, | N 1.71. |

EXAMPLE 26

Synthesis of 3',4'-O-Isopropylidene-6-0-(3-phthalimidopropyl)elsamicin A (8h)

Yield from 6h: 72%
MP 157-160 °C. IR $\nu_{max}$ (KBr) $cm^{-1}$ 3400, 1730, 1700, 1385, 1350, 1240, 1065, 770, 715. UV $\lambda_{max}$ (MeOH) nm ($\epsilon$) 220 (60100), 233 (50800), 266 (41500), 325 (8210), 367 (9270), 390 (1290), 410 (13300). $^1$H NMR ($CDCl_3$ + $D_2O$) $\delta$ 1.38 (3H, d, J = 6 Hz), 1.39 (3H, d, J = 6 Hz), 1.43 (3H, s), 1.45 (3H, s), 1.72 (3H, s) 2.83 (3H, s), 3.37 (3H, s), 2.42 (2H, quint, J = 7 Hz), 5.22 (1H, d, J = 8 Hz), 5.80 (1H, d, J = 4 Hz).

| Anal Calcd for $C_{47}H_{48}N_2O_{15} \bullet H_2O$: | | | |
|---|---|---|---|
| | C 62.80, | H 5.61, | N 3.12. |
| Found: | C 62.83, | H 5.50, | N 3.36. |

EXAMPLE 27

Synthesis of 6-O-(3-Aminopropyl)-3',4'-isopropylideneelsamicin A (8i)

To a solution of 3',4'-O-isopropylidene-6-O-(3-phthalimidopropyl)elsamicin A (8h) (142 mg) in EtOH (1 ml) was added 80% hydrazine hydrate (25 ml) and the reaction mixture was stood at room temperature for 2 hours, diluted with AcOH (20 ml) and evaporated to dryness. The residue was purified by silica gel column chromatography to give 87 mg (72%) of the desired product.
MP 190-199 °C. (dec). IR $\nu_{max}$ (KBr) $cm^{-1}$ 1740, 1360, 1255, 1110, 1070, 1050, 780. UV $\lambda_{max}$ (MeOH) nm ($\epsilon$) 236 (31200), 267 (34500), 311 (5410), 326 (6510), 366 (7580), 391 (10200), 411 (10400). $^1H$ NMR ($CDCl_3$ + $CD_3OD$) $\delta$ 1.2-1.6 (12H, m), 1.72 (3H, s), 2.22 (2H, m), 2.84 (3H, s), 3.40 (3H, s), 5.34 (1H, d, J = 8 Hz), 5.73 (1H, d, J = 4 Hz).
SIMS M/Z: 751 $(M + H)^+$, 392, 334, 200, 160.

**Claims**

**1.** A compound having the formula:

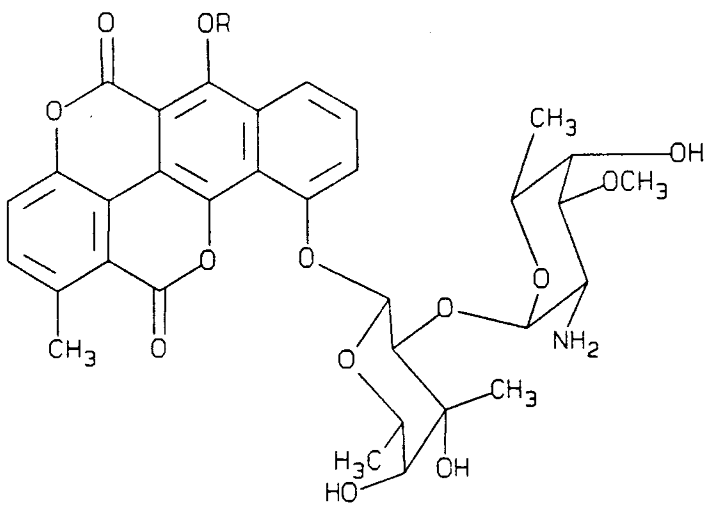

wherein R is $C_1$-$C_{10}$ alkyl or substituted or nonsubstituted amino alkyl.

**2.** A compound having the formula:

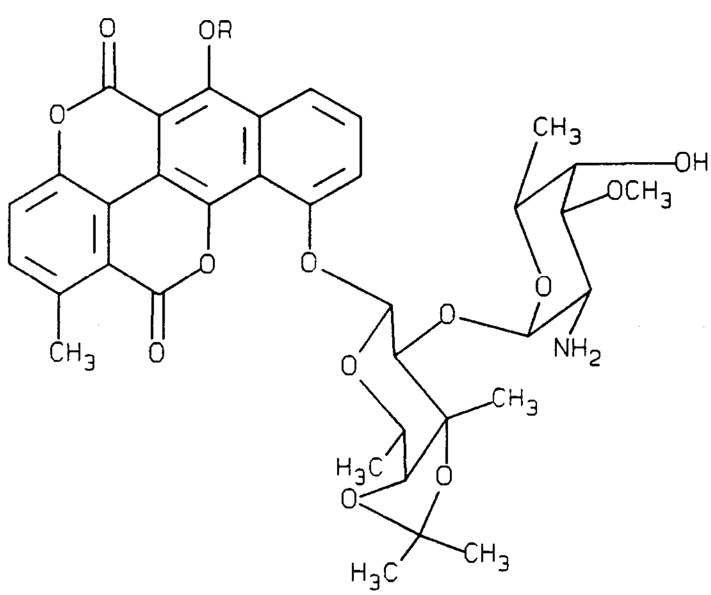

wherein R is $C_1$-$C_{10}$ alkyl or substituted or nonsubstituted amino alkyl.

**3.** The compound of claim 1 which is 6-O-methylelsamicin A;
6-O-n-propylelsamicin A;
6-O-isopropylelsamicin A;
6-O-n-butylelsamicin A;
6-O-n-hexylelsamicin A;
6-O-ethylelsamicin A;
6-O-n-decylelsamicin A;
6-O-(3-phthalimidopropyl)elsamicin A; or
6-O-(3-aminopropyl)elsamicin A;

**4.** The compound of Claim 2 which is 3',4'-O-isopropylidene-6-O-methylelsamicinA;
6-O-ethyl-3',4'-O-isopropylideneelsamcin A;
6-O-n-hexyl-3',4'-O-isopropylideneelsamicin A;
6-O-n-decyl-3',4'-O-isopropylideneelsamicin A;
3',4'-O-isopropylidene-6-O-(3-phthalimidopropyl)elsamicin A; or
6-O-(3-aminopropyl)-3',4'-isopropylideneelsamicin A;

**5.** The intermediates:

2''-N-t-butoxycarbonyl-6-O-methylelsamicin A;

2''-N-t-butoxycarbonyl-6-O-n-propylelsamicin A;

2''-N-t-butoxycarbonyl-6-O-isopropylelsamicin A;

2''-N-t-butoxycarbonyl-6-O-n-butylelsamicin A;

2''-N-t-butoxycarbonyl-6-O-n-hexylelsamicin A;

2''-N-t-butoxycarbonyl-3',4'-O-isopropylidene-6-O-methylelsamicin A;

2''-N-t-butoxycarbonyl-6-O-ethyl-3',4'-O-isopropylideneelsamicin A;

2''-N-t-butoxycarbonyl-6-O-n-hexyl-3',4'-O-isopropylideneelsamicin A;

2''-N-t-butoxycarbonyl-6-O-n-decyl-3',4'-O-isopropylideneelsamicin A; and

2''-N-t-butoxycarbonyl-3,4-isopropylidene-6-O-(3-phthalimidopropyl)elsamicin A.

**6.** A process for preparing a compound as claimed in any one of claims 1 and 3 which comprises alkylating 2''-N-t-butoxycarbonyl-elsamicin A with a suitable alkylating agent, preferably with alkyl halides,if necessary in the presence of a source of iodide and/or a base,to give the corresponding 6-O-alkyl derivatives, and subsequent deprotection, preferably by treating with trifluoroacetic acid.

**7.** A process for preparing a compound as claimed in any one of claims 1 and 3, which comprises alkylating 2''-N-t-butoxycarbonyl-3',4'-O-isopropylideneelsamicin A with a suitable alkylating agent, preferably with alkyl halides, if necessary in the presence of a source iodide and/or a base,to give the corresponding 6-O-alkyl derivatives, and subsequent removal of the isopropylidene group, preferably by treating with trifluoroacetic acid.

**8.** A process for preparing a compound as claimed in any one of claims 2 and 4, which comprises alkylating 2''-N-t-butoxycarbonyl-3',4'-O-isopropylideneelsamicin A with a suitable alkylating agent, preferably with alkyl halides,if necessary in the presence of a source of iodide and/or a base,to give the corresponding 6-O-alkyl derivatives, then treating with an acid, preferably with toluenesulfonic acid.

**9.** A process for preparing 6-O-methylelsamicin A which comprises O-methylation of elsamicin A with

diazomethane.

**10.** A process for preparing 6-O-(3-aminopropyl)-3',4'-isopropylideneelsamicin A which comprises treating 2''-N-t-butoxycarbonyl-3',4'-O-isopropylidenelsamicin A with a 3-phthalimidopropylhalide, if necessary in the presence of a source of iodide and a base,to give the corresponding 6-O-alkyl derivative, then treating with an acid, preferably toluenesulfonic acid and, then treating with hydrazine hydrate.

**11.** A process for preparing 6-O-(3-aminopropyl)elsamicin A which comprises treating 2''-N-t-butoxycarbonyl-3',4'-O-isopropylidenelsamicin A with a 3-phthalimidopropylhalide, if necessary, in the presence of a source of iodide and/or a base,to give the corresponding 6-O-alkyl derivative, then treating with an acid, preferably toluenesulfonic acid,then treating with hydrazine hydrate, and subsequent removal of the isopropylidene group, preferably by treating with trifluoroacetic acid.

**12.** A pharmaceutical formulation which comprises as an active ingredient a compound as claimed in any one of claims 1 - 5, associated with one or more pharmaceutical carriers or diluents.

**13.** A compound as claimed in any one of claims 1 - 5 for use as an antitumor agent.

**Claims for the following Contracting State : ES**

**1.** A process for preparing the compound having the formula:

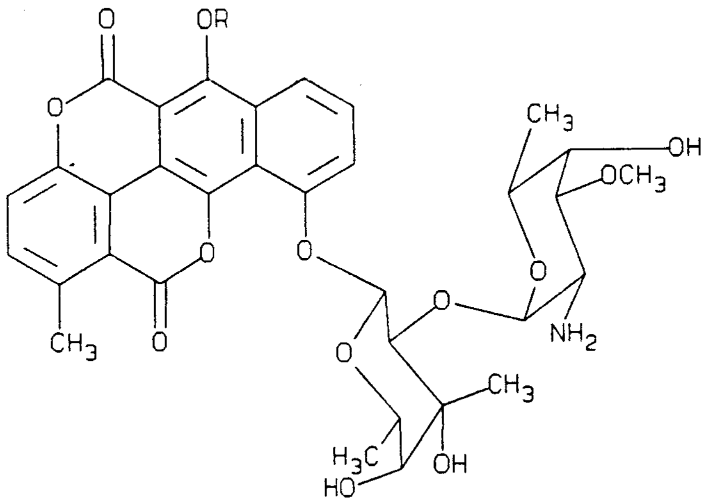

wherein R is $C_1$-$C_{10}$ alkyl or substituted or nonsubstituted amino alkyl, which comprises alkylating 2''-N-t-butoxycarbonyl-elsamicin A with a suitable alkylating agent, preferably with alkyl halides,if necessary in the presence of a source of iodide and/or a base,to give the corresponding 6-O-alkyl derivatives, and subsequent deprotection, preferably by treating with trifluoroacetic acid.

**2.** A process for preparing the compound having the formula:

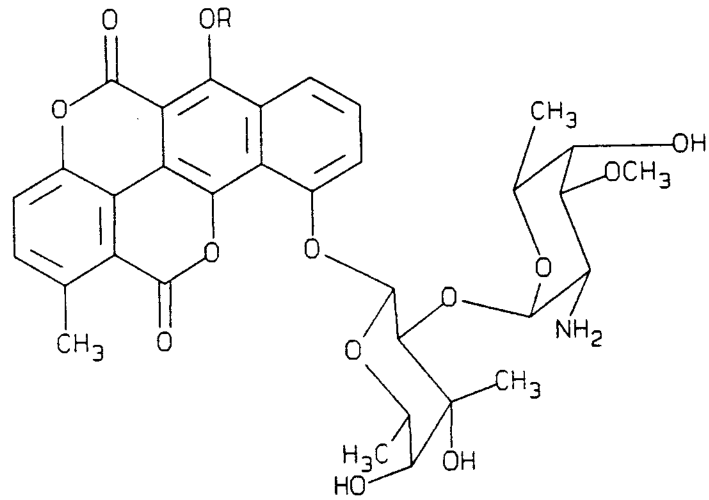

wherein R is $C_1$-$C_{10}$ alkyl or substituted or nonsubstituted amino alkyl, which comprises alkylating 2"-N-t-butoxycarbonyl-3'-4'-O-isopropylideneelsamicin A with a suitable alkylating agent, preferably with alkyl halides, if necessary in the presence of a source iodide and/or a base,to give the corresponding 6-O-alkyl derivatives, and subsequent removal of the isopropylidene group, preferably by treating with trifluoroacetic acid.

**3.** A process for producing the compound having the formula:

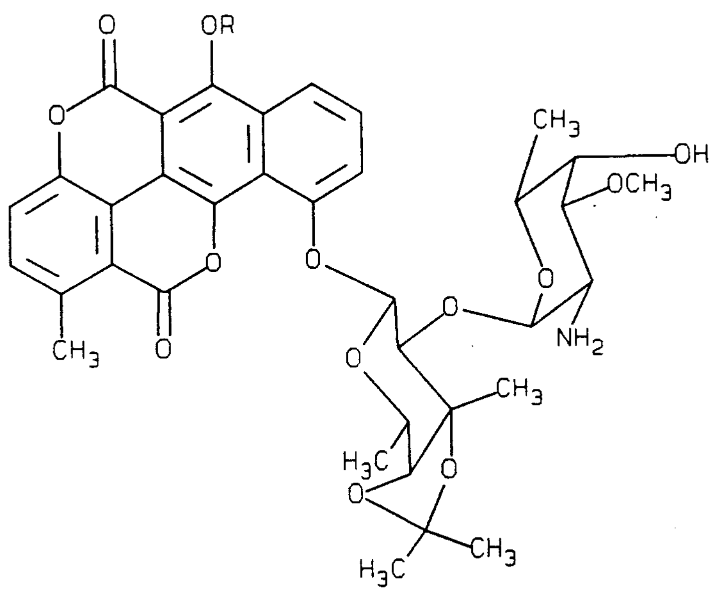

wherein R is $C_1$-$C_{10}$ alkyl or substituted or nonsubstituted amino alkyl, which comprises alkylating 2"-N-t-butoxycarbonyl-3'-4'-O-isopropylideneelsamicin A with a suitable alkylating agent, preferably with alkyl halides,if necessary in the presence of a source of iodide and/or a base,to give the corresponding 6-O-alkyl derivatives, then treating with an acid, preferably with toluenesulfonic acid.

**4.** The process as claimed in any one of claims 1 and 2 for preparing
6-O-methylelsamicin A;
6-O-n-propylelsamicin A;
6-O-isopropylelsamicin A;
6-O-n-butylelsamicin A;
6-O-n-hexylelsamicin A;

6-O-ethylelsamicin A;
6-O-n-decylelsamicin A;
6-O-(3-phthalimidopropyl)elsamicin A;
6-O-(3-aminopropyl)elsamicin A;

**5.** The process as claimed in claim 3 for preparing 3',4'-O-iospropylidene-6-O-methylelsamicin A;

6-O-ethyl-3',4'-O-isopropylideneelsamcin A;
6-O-n-hexyl-3',4'-O-isopropylideneelsamicin A;
6-O-n-decyl-3',4'-O-isopropylideneelsamicin A;
3',4'-O-isopropylidene-6-O-(3-phthalimidopropyl)elsamicin A; or
6-O-(3-aminopropyl)-3',4'-isopropylideneelsamicin A;

**6.** A process for preparing the intermediates

2''-N-t-butoxycarbonyl-6-O-methylelsamicin A.

2''-N-t-butoxycarbonyl-6-O-n-propylelsamicin A.

2''-N-t-butoxycarbonyl-6-O-isopropylelsamicin A.

2''-N-t-butoxycarbonyl-6-O-n-butylelsamicin A.

2''-N-t-butoxycarbonyl-6-O-n-hexylelsamicin A.

which comprises reacting 2''-N-t-butoxycarbonyl-elsamicin A with the appropriate alkylating agent, preferably the appropriate alkyl halide, if necessesary in the presence of a source of iodide and a base.

**7.** A process for preparing the intermediates

2''-N-t-butoxycarbonyl-3',4'-O-isopropylidene-6-O-methylelsamicin A ;

2''-N-t-butoxycarbonyl-6-O-ethyl-3',4'-O-isopropylideneelsamicin A ;

2''-N-t-butoxycarbonyl-6-O-n-hexyl-3',4'-O-isopropylideneelsamicin A

2''-N-t-butoxycarbonyl-6-O-n-decyl-3',4'-O-isopropylideneelsamicin A

2''-N-t-butoxycarbonyl-3,4-isopropylidene-6-O-(3-phthalimidopropyl)elsamicin A

which comprises reacting 2''-N-t-butoxycarbonyl-3',4'-O-isopropyliden-elsamicin A with the appropriate alkylating agent, preferably the appropriate alkyl halide, if necessary in the presence of a source of iodide and a base.

**8.** A process for preparing 6-O-methylelsamicin A, which comprises O-methylation of elsamicin A with diazomethane.

**9.** A process for preparing 6-O-(3-aminopropyl)-3',4'-isopropylideneelsamicin A which comprises treating 2''-N-t-butoxycarbonyl-3'-4'-O-isopropylidenelsamicin A with a 3-phthalimidopropylhalide, if necessary in the presence of a source of iodide and a base,to give the corresponding 6-O-alkyl derivative, then treating with an acid, preferably toluenesulfonic acid and, then treating with hydrazine hydrate.

**10.** A process for preparing 6-O-(3-aminopropyl)elsamicin A which comprises treating 2''-N-t-butoxycarbonyl-3'-4'-O-isopropylidenelsamicin A with a 3-phthalimidopropylhalide, if necessary, in the presence of a source of iodide and/or a base,to give the corresponding 6-O-alkyl derivative, then treating with an acid, preferably toluenesulfonic acid then treating with hydrazine hydrate, and subsequent removal of the isopropylidene group, preferably by treating with trifluoroacetic acid.

**11.** A process for preparing a pharmaceutical composition which comprises admixing a compound of any one of claims 1-5 with one or more pharmaceutical carriers or diluents.

**12.** The use of a compound of any one of claims 1-5 for preparing a pharmaceutical composition for treating or preventing tumors.

**Claims for the following Contracting State : GR**

**1.** A compound having the formula:

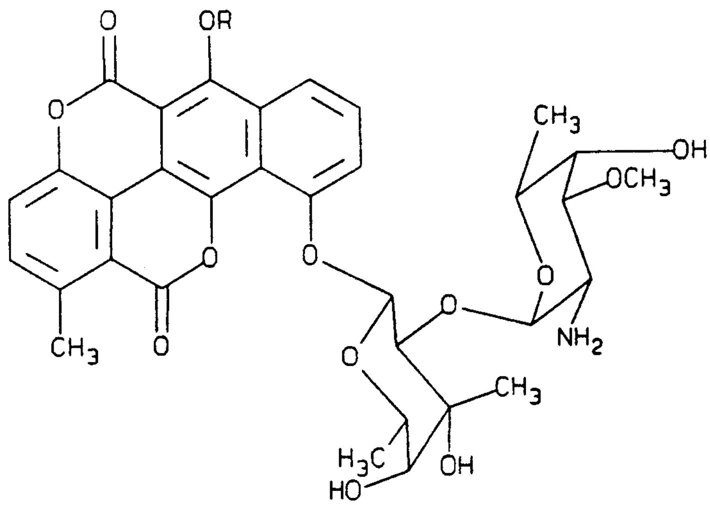

wherein R is $C_1$-$C_{10}$ alkyl or substituted or nonsubstituted amino alkyl.

**2.** A compound having the formula:

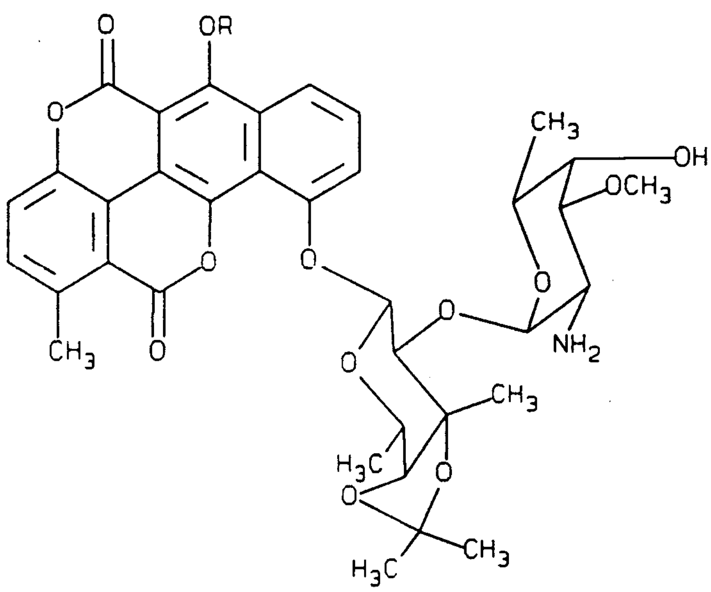

wherein R is $C_1$-$C_{10}$ alkyl or substituted or nonsubstituted amino alkyl.

**3.** The compound of claim 1 which is 6-O-methylelsamicin A;
6-O-n-propylelsamicin A;
6-O-isopropylelsamicin A;

6-O-n-butylelsamicin A;
6-O-n-hexylelsamicin A;
6-O-ethylelsamicin A;
6-O-n-decylelsamicin A;
6-O-(3-phthalimidopropyl)elsamicin A; or
6-O-(3-aminopropyl)elsamicin A;

**4.** The compound of Claim 2 which is 3',4'-O-isopropylidene-6-O-methylelsamicinA;
6-O-ethyl-3',4'-O-isopropylideneelsamcin A;
6-O-n-hexyl-3',4'-O-isopropylideneelsamicin A;
6-O-n-decyl-3',4'-O-isopropylideneelsamicin A;
3',4'-O-isopropylidene-6-O-(3-phthalimidopropyl)elsamicin A; or
6-O-(3-aminopropyl)-3',4'-isopropylideneelsamicin A;

**5.** The intermediates:

2''-N-t-butoxycarbonyl-6-O-methylelsamicin A;

2''-N-t-butoxycarbonyl-6-O-n-propylelsamicin A ;

2''-N-t-butoxycarbonyl-6-O-isopropylelsamicin A;

2''-N-t-butoxycarbonyl-6-O-n-butylelsamicin A;

2''-N-t-butoxycarbonyl-6-O-n-hexylelsamicin A;

2''-N-t-butoxycarbonyl-3',4'-O-isopropylidene-6-O-methylelsamicin A;

2''-N-t-butoxycarbonyl-6-O-ethyl-3',4'-O-isopropylideneelsamicin A;

2''-N-t-butoxycarbonyl-6-O-n-hexyl-3',4'-O-isopropylideneelsamicin A;

2''-N-t-butoxycarbonyl-6-O-n-decyl-3',4'-O-isopropylideneelsamicin A; and

2''-N-t-butoxycarbonyl-3,4-isopropylidene-6-O-(3-phthalimidopropyl)elsamicin A.

**6.** A process for preparing a compound as claimed in any one of claims 1 and 3 which comprises alkylating 2''-N-t-butoxycarbonyl-elsamicin A with a suitable alkylating agent, preferably with alkyl halides if necessary in the presence of a source of iodide and/or a base,to give the corresponding 6-O-alkyl derivativeS, and subsequent deprotection, preferably by treating with trifluoroacetic acid.

**7.** A process for preparing a compound as claimed in any one of claims 1 and 3, which comprises alkylating 2''-N-t-butoxycarbonyl-3',4'-O-isopropylideneelsamicin A with a suitable alkylating agent, preferably with alkyl halides, if necessary in the presence of a source iodide and/or a base,to give the corresponding 6-O-alkyl derivatives, and subsequent removal of the isopropylidene group, preferably by treating with trifluoroacetic acid.

**8.** A process for preparing a compound as claimed in any one of claims 2 and 4, which comprises alkylating 2''-N-t-butoxycarbonyl-3',4'-O-isopropylideneelsamicin A with a suitable alkylating agent, preferably with alkyl halides,if necessary in the presence of a source of iodide and/or a base,to give the corresponding 6-O-alkyl derivatives, then treating with an acid, preferably with toluenesulfonic acid.

**9.** A process for preparing 6-O-methylelsamicin A which comprises O-methylation of elsamicin A with diazomethane.

**10.** A process for preparing 6-O-(3-aminopropyl)-3',4'-isopropylideneelsamicin A which comprises treating 2''-N-t-butoxycarbonyl-3',4'-O-isopropylidenelsamicin A with a 3-phthalimidopropylhalide, if necessary in the presence of a source of iodide and a base,to give the corresponding 6-O-alkyl derivative, then

treating with an acid, preferably toluenesulfonic acid and, then treating with hydrazine hydrate.

**11.** A process for preparing 6-O-(3-aminopropyl)elsamicin A which comprises treating 2''-N-t-butoxycarbonyl-3',4'-O-isopropylidenelsamicin A with a 3-phthalimidopropylhalide, if necessary, in the presence of a source of iodide and/or a base,to give the corresponding 6-O-alkyl derivative, then treating with an acid, preferably toluenesulfonic acid then treating with hydrazine hydrate, and subsequent removal of the isopropylidene group, preferably by treating with trifluoroacetic acid.

**12.** A process for preparing a pharmaceutical formulation which comprises admixing a compound as claimed in any one of claims 1-5, with one or more pharmaceutical carriers or diluents.

**13.** A compound as claimed in any one of claims 1-5 for use as an antitumor agent.

European Patent Office

# EUROPEAN SEARCH REPORT

Application Number

EP 92 10 9112

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 219 852 (ISHIHARA SANGYO KAISHA LTD.) * abstract * --- | 1,2,13 | C07H17/04 A61K31/70 |
| D,A | US-A-4 518 589 (M.KONISHI ET AL.) * claims 1-8 * ----- | 1,2,13 | |

TECHNICAL FIELDS SEARCHED (Int. Cl.5)

C07H
A61K

The present search report has been drawn up for all claims

| Place of search | Date of completion of the search | Examiner |
|---|---|---|
| THE HAGUE | 29 JULY 1992 | SCOTT J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)